# EUROPEAN PATENT APPLICATION

(11) **EP 3 868 401 A1**
(43) Date of publication of application: **25.08.2021**
(21) Application number: 20305173.5
(22) Date of filing: 21.02.2020
(51) Int. Cl.: A61K 39/385, A61P 37/04

(54) **OPTIMIZATION OF PEPTIDE-MELANIN BINDING**

(71) Applicant: Assistance Publique Hôpitaux de Paris, 75004 Paris (FR); Altevax, 75116 Paris (FR)
(72) Inventor: CARPENTIER, Antoine, 75007 PARIS (FR); BANISSI, Claire, 94300 VINCENNES (FR)
(74) Representative: Flesselles, Bruno F.G.

(57) **Abstract**

The invention relates to the use of melanin, complexed with a peptide, in particular containing epitopes for use as an immunostimulatory composition, wherein the peptide that has been modified as to increase nucleophilicity.

## Description

The invention is in the field of peptide delivery, in particular for immunological purposes, notably in the field of adjuvants, *i.e.* elements that potentiate the immunogenic property of antigens, and is useful in particular in the field of vaccines, whether prophylactic or therapeutic.

Melanin is a pigment, obtained by oxidative polymerization of precursors.

Melanin is widely found in the animal kingdom, especially but not exclusively in the skin, and several functions have been attributed to melanin (d'Ishia et al 2015), among which:
- Photoprotection against mutagenic light which is one of its most important biological functions.
- Protection against oxidative stress (free radical scavenger)
- Hair and skin pigmentation
- Innate immunity in insects
- Metal homeostasis

Grafting biologically active molecules such as peptides, proteins, glycoproteins, or lipids on melanin can be useful to take advantages of the biological and physicochemical properties of melanin.
- Melanin can protect the bound molecule from UV light, degradation by chemical compounds or enzymes
- Melanin absorbs light, and can thus selectively heat the bound molecules
- When injected in the body (for examples subcutaneously), melanin makes a depot effect that can allow a local release. Melanin, once injected, is also partially driven to the draining lymph nodes, and can thus be used as a carrier for a molecule to reach the lymphatic system. These properties can be particularly useful in vaccine approaches as disclosed by Carpentier et al (PLoS One. 2017 Jul 17;12(7):e0181403) and WO2017089529).

Historical vaccines were based on live attenuated pathogens, whole inactivated organisms, or modified toxins. To limit potential side-effects, recent developments have focused on subunit vaccines which are generally composed of 30-60 amino acids but can be limited to one epitope as short as 8 amino acids. The use of a small portion of an antigen limits the risk of potential cross-reactivity by focusing the immune response against the desired portion of an antigen. However, subunit vaccines, and especially peptide subunit vaccines, are often poorly immunogenic, due to the lack of pathogen-derived molecules to act as danger signals. Subunit vaccines thus require additional adjuvants to be effective (Fujita et al, Chem Cent J. 2011;5(1):48; Azmi et al, Hum Vaccin Immunother. 2014;10(3):778-96).

Despites all the progress made, several limitations are still faced by modern vaccines. Subunit antigens are often poorly immunogenic. The dose of antigen required to trigger the immunity (usually within the range of 10 to 300µg) might be a limiting factor, especially when antigen is difficult to manufacture, or when demand exceeds production capacity. Moreover, induction of CD8+ responses remains a difficult challenge because extracellular molecules are usually presented by MHC class II and not by MHC class I. Finally, vaccine formulations, such as emulsion, liposomes, fusion molecules can be either unstable or difficult to synthesize, making the cost of manufacturing sometimes prohibitive. The ideal adjuvant should thus be potent to trigger or boost an Ag-specific immune response (both humoral and cellular responses), easy to manufacture, non toxic, and stable.

The melanin pigment is known and recognized in the art and differs from a mere polypeptides of amino-acids that are melanin precursors. For instance, a polytyrosyl or a polydopa peptide, obtained by protein synthesis (linking the N-terminus of an amino acid to the C-terminus of another amino acid), without oxidative polymerization, is not a melanin molecule and would not be considered as such by a person skilled in the art.

Melanin is thus a broad and generic term for designating a group of natural pigments found in most organisms, produced by the oxidation of the amino acid tyrosine (or another precursor), followed by polymerization. This oxidation, which is a critical step, is generally mediated by the enzyme tyrosinase, which will convert tyrosine to DOPA.

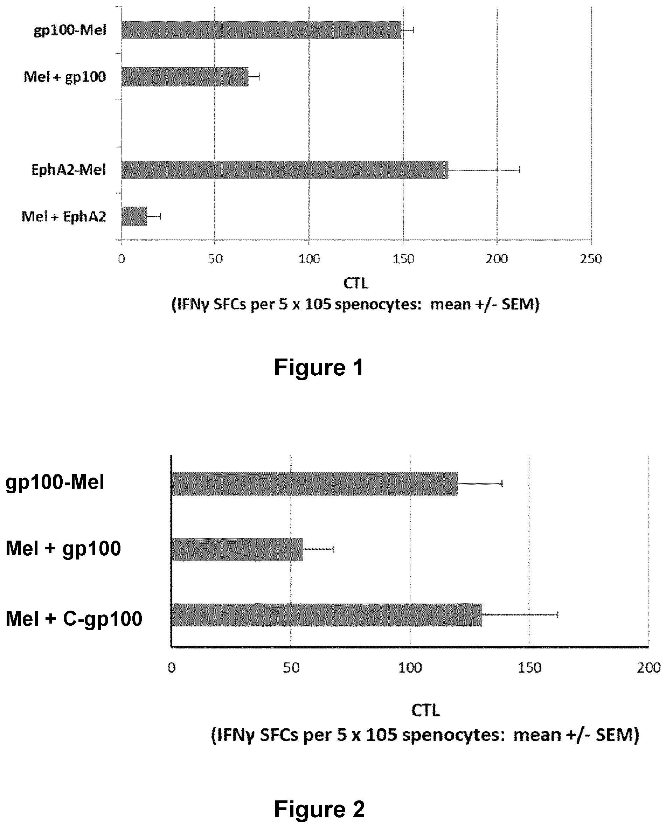

Melanin is thus obtained through a complex process (as reminded in Figure 1) that combines both the oxidation of melanin precursors and their subsequent polymerization.

Melanin is naturally present in a lot of organisms and can also be synthetically produced (oxidative polymerization *in vitro*) and is sold as such, for instance by Sigma Aldrich, as prepared oxidation of tyrosine with hydrogen peroxide.

Melanin synthesis involves several intermediary compounds, several enzymes and can be modified by pH, presence of cationic metals, temperature.

As intermediary compounds, one could cite: L-phenylalanine, L-tyrosine, L-dopa, dopaquinone, cyclodopa, dopachrome, quinone methide, benzothiazole, benzothiazine, dihydroesculetin, Dihydroxyindole carboxylic acid (DHICA), 5, 6-dihydroxyindole (DHI), dopamine-o-quinone, Dopamine Leukodopaminochrome, dopaminochrome, norepinephrine, noradenochrome, epinephrine, adenochrome, 3-amino-tyrosine, and others.

As enzymes involved in the synthesis, one could cite: Phenylalanine hydroxylase, tyrosinase (EC 1.14.18.1 and EC1.10.3.1), mushroom tyrosinase, tyrosine hydroxylases, peroxidase, Phenol-oxidase, Dopachrome tautomerase (E.C.5.3.2.3, DCT/Trp2); DHICA oxidase (Trp1) DHI oxidase.

A synthetic melanin is the product of *in vitro* oxidative polymerization of a melanin precursor. Such polymerization is performed in the presence of an oxidant. Such melanin could be differentiated from natural melanin as described above, as it would be a bit more homogeneous.

Arnon et al (1960 - Biochem. J., 75: 103-109) disclose among others, antigen protein, i.e. gelatin, egg albumin or edestin, bound to polytyrosyl, which is not a melanin.

Sela et al (Biochem. J., vol. 75, 1 January 1960 (1960-01-01), pages 91-102) disclose an operating process for obtaining polypeptidyl gelatin. There is no oxidative polymerization and the obtained product does not comprise melanin and cannot be considered as a melanin.

Akagi et al (In: "Bioactive Surfaces", 1 January 2011 (2011-01-01), Springer Berlin Heidelberg, Berlin, Heidelberg, Adv Polym Sci, vol. 247, pages 31-64) disclose Biodegradable Nanoparticles as Vaccine Adjuvants and Delivery Systems. Polyaminoacid nanoparticles are prepared with tyrosin, but this document does not disclose nor mention melanin or that the polymerization would give melanin as the final product.

US 2004/057958 discloses an immunogenic carrier which can be a polyamino acid polymer. This document never mentions or suggests using melanin as an immunogen.

Fujita et al (Chemistry Central Journal, Biomed Central Ltd, vol. 5, no. 1, 23 August 2011 (2011-08-23), page 48) reviews the status of multiple antigen-presenting peptide vaccine systems, using nanoparticles. This document does not mention nor suggest preparing complexes of melanin and antigens for increasing immunogenicity of the antigen.

Cui et al (Biomacromolecules, vol. 13, no. 8, 13 August 2012 (2012-08-13), pages 2225-2228) describes use of polydopamine films on capsule to perform intracellular drug delivery. The particles are different from melanin and not used to obtain an immunogenic composition.

Cui et al (NANO, vol. 10, no. 05, 1 July 2015 (2015-07-01), pages 1530003-1 to 1530003-23) further disclose poly-dopamine capsules. The particles are different from melanin and not used to obtain an immunogenic composition.

Lee et al (Advanced Materials, vol. 21, no. 4, 26 January 2009 (2009-01-26), pages 431-434) disclose that polydopamine films can be bioconjugated to various substrates, are different from melanin and do not indicate that these films display immunogenic properties.

Park et al (ACS Nano, vol. 8, no. 4, 22 April 2014 (2014-04-22), pages 3347-3356) disclose polydopamine nanoparticles used for carrying drugs. The particles are different from melanin. This document does not mention or suggest melanin-antigen complexes as immunogenic compositions.

US 2012/237605 discloses nanoparticles with a polydopamine-based surface, but does not suggest or disclose the use thereof as immunogenic compositions. The particles are different from melanin.

Liu et al (Small. 2016 Apr 6;12(13):1744-57) disclose pathogen-mimicking polymeric nanoparticles coated with polydopamine as vaccines adjuvants induce robust humoral and cellular immune responses.

WO2017089529 discloses the use of a melanin, complexed with an antigen, as an immunostimulatory composition. Such compositions are obtained by performing the oxidative polymerization of the melanin precursor in the presence of the antigen. In this document, the described antigens are peptides harboring T-cell epitopes such as the human gp100 epitope. These are used as a vaccine to protect an animal against a disease implicating (i.e. involving and/or concerning) cells expressing inside the cells, at their surface, or secreting such target antigen or epitopes thereof. Complexing the antigen with the melanin makes it possible to improve the immune response.

Slominski et al (Physiol Rev. 2004 Oct;84(4):1155-228) and Micillo et al (Int J Mol Sci. 2016 May 17;17(5). pii: E746) describe pheomelanogenesis, as an alternative pathway in melanogenesis where cysteine or glutathione (containing cysteine) binds to dopaquinone to yield cysteinyldopa and glutathionyldopa which is then transformed into pheomelanin. This binding of cysteine to a melanin precursor thus occurs before pheomelanin has been synthetized. Furthermore, addition of cysteine before polymerization as a free amino acid is not intended to help the binding of peptides to the melanin.

The Applicant has determined that it is possible to increase the immune response against an antigen by complexing such antigen with a melanin already formed. In contrast to the disclosure of WO2017089529, where the antigen was added prior to the oxidative polymerization, such effect is also observed when the antigen is added to the melanin prior to its formation by oxidative polymerization. Surprisingly, the Applicant showed that the binding of the antigen to the melanin is increased by addition of one or several amino-acids containing a nucleophilic residue to the antigen, thereby increasing the biological activity (immune response) as compared to when a peptide without such addition is used.

The invention thus relates to a method for obtaining a composition, or for binding a peptide to a synthetic melanin, comprising the steps of
a) Providing a synthetic melanin which has been obtained by an oxidative polymerization of a melanin precursor, and
b) Mixing it with a peptide that has been modified by the addition of one or several amino-acids containing a nucleophilic residue so as to allow or increase the binding of the peptide to melanin.

In a specific embodiment, the peptide is an immunologically active peptide and the invention thus makes it possible to obtain an immunostimulatory composition, comprising the steps of
a) Providing a synthetic melanin which has been obtained by an oxidative polymerization of one or several melanin precursors, and
b) Mixing it with an immunologically active peptide that has been modified by the addition of one or several amino-acids containing a nucleophilic residue
thereby obtaining a composition where the peptide is bound to melanin. In this method, the binding of the peptide to melanin is increased as compared to a peptide which has not been modified. Furthermore, by increasing the binding to the melanin, biological activity of the peptide is increased when the composition is administered to a subject, as compared to the activity observed when a peptide which has not been modified has been added to the melanin.

As described therein, an "immunostimulatory composition" is a composition containing at least one antigen and that induces an immune response against an epitope of such antigen after administration to a host. Said host is a human or an animal and is preferably a human being.

As defined herein, a "synthetic melanin" is a melanin pigment (or macromolecule) obtained *in vitro* by oxidative polymerization of a melanin precursor.

### Nucleophilic amino acids

The invention thus relates to the introduction of a modification in the sequence of a peptide so as to add nucleophilic amino acids thereto. Consequently, the sequence of the resulting peptide presents a chain of amino acid that is not present in the native peptide or in the native protein from which the peptide has been isolated. This resulting peptide is thus a new entity and is different from peptides existing in the art.

By nucleophilic amino acid, it is intended to design an amino acid presenting a nucleophilic moiety. Nucleophilicity is a measure of how rapidly molecules with lone pairs of electrons can react in nucleophilic substitution reactions. The terminal NH2 moiety of a peptide and some of the side chains of its amino-acids are known nucleophiles. As amino acids with nucleophilic side chains, one can cite Cys (RSH, pKa 8.5-9.5), His (pKa 6-7), Lys (pKa 10.5) and, to a minor degree, Ser (ROH, pKa 13) or Methionine. In addition, proline or hydroxyproline, which can be added to the NH2-terminus of a peptide, are nucleophiles.

In a preferred embodiment, an amino acid comprising a side chain comprising a NH or a NH2 moiety, or a sulfur atom, is added preferably to the N-terminus of the peptide in order to make such a nucleophilic modification.

In this embodiment, the residue added to the peptide is selected from the group consisting of cysteine, acetylcysteine, proline, hydroxyproline, lysine, and histidine.

In another embodiment, the residue added to the peptide is selected from the group consisting of cysteine, hydroxyproline and lysine.

Addition of Cysteine to the N-terminus of a peptide is particularly preferred, in particular with the peptides described herein as SEQ ID NO: 22, SEQ ID NO: 23, SEQ ID NO: 24, SEQ ID NO: 25, SEQ ID NO: 26, and SEQ ID NO: 27.

### Peptide to modify

The peptide that is modified and bound to the melanin is preferably a biologically or immunologically active peptide.

It preferably presents at least 3 amino acids, more preferably at least 8 amino acids. It contains generally at most 100 amino acids, more preferably at most 50 amino acids, more preferably at most 40 amino acids. A peptide containing between 8 and 50 amino acids is thus perfectly suitable for modification and use according to the methods herein disclosed.

Biologically active (or bioactive) peptides are peptides that interact with proper body receptors, and provide a beneficial or detrimental effect. Examples of such peptides can be found in Kastin and Pan (Curr Pharm Des. 2010;16(30):3390-3400) or in Iwaniak and Minkiewicz (Polish Journal of Food and Nutrition Sciences, 2008. 58. 289-294). One can cite coeliac toxic peptides, such as fragments of gliadins or prolin-rich peptides, immunomodulating peptides, including glycopeptides, hormones, peptidic fragments of immunoglobulins and peptides isolated from food proteins (Werner, Immunol Lett. 1987 Dec;16(3-4):363-70), such as oryzatensin, peptides isolated from casein and whey proteins from human and bovine milk, opioid and opioid agonist peptides.

An immunologically active peptide is a peptide that is capable of inducing an immune response (preferably in human or mammals) which is cross reactive with an antigen and preferably presents a protective effect against such antigen. In some embodiment, an immunologically active peptide is a peptide isolated from a protein antigen. An immunologically active peptide thus contains one or more epitopes of an antigen, preferably at least one T-cell epitope. In particular, an immunologically active peptide is selected from the group consisting of SEQ ID NO: 22, SEQ ID NO: 23, SEQ ID NO: 24, SEQ ID NO: 25, SEQ ID NO: 26 and SEQ ID NO: 27.

### Antigen

In the context of the invention, an "antigen" is a molecule or a combination of molecules against which it is desired to elicit an immune response in order for the immune system of a living animal to recognize it. Such antigen may be foreign to the body of the host to which the immune response is sought. In this case, the antigen may be a protein expressed by a bacteria, a parasite, a fungus or a virus. The antigen may also be a self-antigen, *ie* a protein that is expressed by cells of the host, such as tumor antigens.

Antigens can consist of whole organisms (viruses or bacteria, fungi, protozoa or even cancer cells), killed or not, cells (irradiated or not, genetically modified or not), or fractions of these organisms/cells like cell extracts or cell lysates. Antigens can also consist of single molecules like proteins, peptides, polysaccharides, lipids, glycolipids, glycopeptides or mixture thereof. Antigens may also be one of the above-cited molecules that has been modified through a chemical modification or stabilization. In particular, the net charge of the antigen can be modified using adequate substitution of amino acids or chemical modifications of the antigen.

An antigen may be a full protein, or any part of a protein, such as an epitope of the protein. The peptide designed to elicit a response against an antigen, in the context of the present invention may consist in a synthetic peptide or molecule that contains multiple epitopes that are linked together. In an embodiment, these epitopes are specific of a MHC haplotype.

In another embodiment, the peptide may contain multiple epitopes obtained from various antigens of the same pathogen (the term pathogen preferably indicate a foreign pathogenic agent such as a bacteria, a virus, a parasite or a fungus, but may also extend to tumour cells). In this case, one can use the immunostimulatory molecule to obtain a strong immune response against this pathogen.

The peptide that can be used with the melanin macromolecule in the disclosed composition may contain sequences of any antigen against which an immune response is searched.

This antigen, before modification, can be a full protein as found in nature, and is preferably only part of a protein found in nature.

The peptide as intended in the immunogenic composition as herein described, can also be a mixture of peptides.

The antigen may be a protein, a peptide, a polysaccharide, or a lipid. The antigen may be part (coats, capsules, cell walls, flagella, fimbrae, and toxins) of a bacteria, a virus, or another microorganisms. The antigen may be a more complex molecule such as a lipid combined with a protein and/or a polysaccharide.

### Epitopes

In a particular embodiment, the peptide as used in the immunogenic composition comprises one or several MHC epitopes.

In a particular embodiment, the peptide as used in the immunogenic composition contains a single MHC epitope, or consists in a single MHC epitope.

In another embodiment, the peptide as used in the immunogenic composition contains one or several MHC epitopes, flanked, at its N and/or C terminus by a few amino-acids (between 1 and 10, preferably between 1 and 6 amino-acids at one, or both C and N terminal ends).

MHC epitopes (or T cell epitopes) are presented on the surface of an antigen-presenting cell, where they are bound to MHC molecules. T cell epitopes presented by MHC class I molecules are typically peptides between 8 and 11 amino acids in length, whereas MHC class II molecules present longer peptides, 13-17 amino acids in length (https://en.wikipedia.org/wiki/Epitope#T_cell_epitopes).

The MHC epitope may be synthetized *in vitro* (with or without addition of amino acids at its C and/or N terminal extremities). MHC bound peptides may be extracted from live cells, in particular tumor cells, by any method known in the art such as acid treatment in particular with hydrochloric acid.

In another embodiment, the peptide comprises one or several B cell epitopes, *i.e.* part of a protein that is recognized by an antibody, preferably linear epitopes, formed by a continuous sequence of amino acids from the antigen.

In a particular embodiment, the peptide as used in the immunogenic composition consists in a B cell epitope.

In another embodiment, the peptide as used in the immunogenic composition consists in a B cell epitope which is flanked, at its N and/or C terminus by a few amino-acids (between 1 and 10, preferably between 1 and 6 amino-acids at one, or both C and N terminal ends).

Different methods in the literature, relating to epitope mapping, make it possible to identify T cell or B cell epitopes from a given antigen.

The composition as disclosed herein rather uses antigenic epitopes, rather than the full antigens. Using only epitopes (*i.e.* small antigenic parts) to elicit an immune response is particularly interesting to limit any adverse effects that could be associated with the use of large size proteins.

### Other synthetic antigens

In particular, the peptide may be a synthetic molecule comprising multiple epitopes, separated by stretches of amino acids or any other acceptable linkers such as polyether compounds or other linkers used in dendrimer constructs (Tam, Proc Natl Acad Sci USA. 1988, 85(15):5409-13; Seelbach et al, Acta Biomater. 2014 10(10):4340-50; Sadler and Tam, Reviews in Molecular Biotechnology 90, 3-4, pp195-229; Bolhassani et al, Mol Cancer. 2011 Jan 7;10:3).

The multiple epitopes may be epitopes specific for different HLA haplotypes (in order to generate a single immunogenic or immunostimulatory composition that able to elicit a immune response against a given antigen or pathogen in a broad population of patients.

In another embodiment, the epitopes may originate from the same or multiple antigens of the same pathogenic agent, in order to elicit a strong immune response against said pathogenic having the multiple epitopes.

In another embodiment, the epitopes may originate from different pathogenic agents, in order to elicit an immune response against these various agents at one time, by using the immunogenic composition.

The antigen may contain universal T helper epitopes such as pan-DR epitope (PADRE) and Pol₇₁₁ epitopes. The literature widely discloses other universal T helper epitopes.

### Source of the antigens

Antigens that can be used in the present invention can be chosen in particular among:
- Exogenous antigens (antigens that have entered the body from the outside, for example by inhalation, ingestion or injection; these antigens are generally presented by MHC II molecules).
- Endogenous antigens (antigens generated within normal cells as a result of normal cell metabolism, or because of viral or intracellular bacterial infection; these antigens are generally presented by MHC I molecules).
- Neoantigens (such as tumor antigens, such as epitopes derived from viral open reading frames in virus-associated tumors, or other tumor antigens presented by MHC I or MHC II molecules on the surface of tumor cells.
- Allergens (an antigen capable of stimulating a type-I hypersensitivity reaction in atopic individuals through Immunoglobulin E (IgE) responses).

As examples of tumor antigens, one can cite alphafetoprotein (AFP) found in germ cell tumors and hepatocellular carcinoma, carcinoembryonic antigen (CEA) found in bowel cancers, CA-125 found in ovarian cancer, MUC-1 found in breast cancer, epithelial tumor antigen (ETA) found in breast cancer, tyrosinase or melanoma-associated antigen (MAGE) found in malignant melanoma, abnormal products of ras, p53 found in various tumors, gp100 (Melanocyte protein PMEL, a type I transmembrane glycoprotein enriched in melanosomes), TRP2 (Tyrosinase-Related Protein 2), EPHA2 (receptor tyrosine kinase, frequently overexpressed in a wide array of advanced cancers), NY-ESO-1, survivin (baculoviral inhibitor of apoptosis repeat-containing 5 or BIRC5, expressed in particular in breast and lung cancer), Brevican core protein, Chitinase-3-like protein 1 or 2, Fatty acid-binding protein, Brain Elongation of very long chain fatty acids protein 2, Receptor-type tyrosine-protein phosphatase zeta, Telomerase reverse transcriptase (TERT), EGFRvIII (epidermal growth factor receptor mutant, expressed in particular in glioblastomas).

As examples of pathogens from which antigens can be used in the immunogenic composition, one can cite any pathogens involved in infectious diseases (virus, bacteria, parasite, mycosis).

For infectious diseases, preferred pathogens are selected from human immune deficiency virus (HIV), hepatitis A and B viruses, hepatitis C virus (HCV), Rous sarcoma virus (RSV), Ebola viruses, Papovavirus, Coronavirus, Papillomavirus, Cytomegalovirus, Herpes viruses, Varicella Zoster Virus, Epstein Barr virus (EBV), Influenza virus, Adenoviruses, Rotavirus, Rubeola and rubella viruses, Variola virus, *Staphylococcus, Chlamydiae, Mycobacterium tuberculosis, Streptococcus pneumoniae, Bacillus anthracis, Vibrio cholerae, Helicobacter Pilorii, Salmonella, Plasmodium* sp. (*P. falciparum, P. vivax,* etc.), *Pneumocystis carinii, Giardia duodenalis* , *Schistosoma* (Bilharziose), Leishmania, *Aspergillus, Cryptococcus, Candida albicans, Listeria monocytogenes,* or *Toxoplasma gondii.*

As examples of diseases which can benefit from immunizations with an appropriate antigen one can cite: cancer (benign or malignant tumors); hematological malignancies, allergies, autoimmune diseases, chronic diseases such as atherosclerosis, or Alzheimer disease.

The antigen is thus preferably a bacterial or viral antigen (or a polypeptide or polymer (such as the ones usable in dendrimers) containing one or more epitopes isolated from a bacterial or viral antigen).

In another embodiment, the antigen is a self-antigen (endogenous or neoantigen), in particular a tumor specific antigen (or a polypeptide containing one or more epitopes isolated from such antigens).

In another embodiment, the antigen is an allergen or a polypeptide containing one or more epitopes isolated from such antigen.

An antigen is said to be associated with the disease when said antigen is present specifically during the course of the disease. Such antigens are thus bacterial, viral, fungal or parasitic antigens in case of infectious diseases, or tumor antigens in case of cancer diseases.

In particular, the peptide is selected from the group consisting of SEQ ID NO: 22 (YAVGYMLRLGAPASKL), especially useful for treating glioma, SEQ ID NO: 23 (TTMDQKSLWAGVVVLL), especially useful for treating glioma, SEQ ID NO: 24 (KSVWSKLQSIGIRQH), especially useful for treating bladder cancer, urinary tract cancer or liposarcoma, SEQ ID NO: 25 (YVGYLQPRTFLLKYN), SEQ ID NO: 26 (GYLQPRTFLLK) and SEQ ID NO: 27 (KVVNQNAQAL), all last three usable for treating coronavirus Covid-19. Such peptides are to be modified according to the teachings of the present document, by addition of a nucleophilic amino acid in their sequence, preferably at the N-terminal extremity. It is preferred when the added amino acid is cysteine.

### Obtaining the synthetic melanin

The synthetic melanin is obtained after oxidative polymerization of melanin precursors *in vitro.*

Polymerization of melanin precursors can be performed by methods known in the art. In particular, the melanin precursor may be incubated, with or without buffer, with an enzyme such as phenylalanine hydroxylase, tyrosinase, mushroom tyrosinase, tyrosine hydroxylase, peroxidase, Phenol-oxidase, Dopachrome tautomerase, DHICA oxidase, DHI oxidase. The choice of the enzyme will be made by the person skilled in the art depending on the nature of the precursor present in solution before polymerization.

The mixture is also exposed to an oxidizing agent as disclosed above in order to promote the polymerization and obtain the synthetic melanin.

Among others, the person skilled in the art may optimize various parameters such as the ratio of melanin precursors is a mixture is used, the type of oxidant, pH, buffer, length of incubation, or temperature of reaction.

In particular, melanin synthesis may be influenced by pH (alkaline pH promoting auto-oxidation of catechol), and presence of metal ions (such as Cu²⁺, Ni²⁺, Fe³⁺, Fe²⁺, Co²⁺, Zn²⁺, Mn²⁺, Mg²⁺...) present in the incubation solution (Palumbo et al, Biochim Biophys Acta. 1987; 13;925(2):203-9; Palumbo et al, Biochim Biophys Acta. 1991;1115(1):1-5; WO95009629).

### Melanin Precursor

A "melanin precursor" is a molecule that is used or synthetized during the synthesis of a melanin *in vitro.* In particular, one can cite: L-phenylalanine, L-tyrosine, L-dopa, dopaquinone, cyclodopa, dopachrome, Dihydroxyindole carboxylic acid or 5,6-dihydroxyindole-2carboxylic acid (DHICA), indol 5,6 quinone, 5,6-dihydroxyindole (DHI), dopamine-o-quinone, Dopamine leukodopaminochrome, leukodopachrome (cyclodopa), dopaminochrome, norepinephrine, noradequinone, noradenochrome, epinephrine, epinephrine-o-quinone, adenochrome, 3-amino-tyrosine, 6-hydroxy-Dopa, dihydrocaffeic acid, caffeic acid and others.

The term "melanin precursor" further includes derivatives of such precursors and/or polymers containing a high proportion of such precursors (such as in Mussel Adhesives Proteins). Such melanin precursors and derivatives are described in WO2017089529 and can be used as equivalent melanin precursors in the context of the present invention.

The melanin precursor is preferably selected from the group consisting of DHICA, DHI, L-dopa, L-tyrosine, D-dopa, 6-hydroxy-Dopa, dopaquinone, cyclodopa, dopachrome, dopamine-o-quinone, dopamine, leukodopaminochrome and dopaminochrome.

A preferred melanin precursor is L-dopa. Another preferred melanin precursor is DHICA. Another preferred melanin precursor is DHI. Another preferred melanin precursor is L-tyrosine. In a specific embodiment, the melanin precursor is a mixture of DHICA and DHI. In another embodiment, the melanin precursor is dopachrome.

### Oxydizing agent

An "oxidizing agent" or "oxidizing molecule" is a compound that is able to promote oxidative polymerization of a solution containing melanin precursors and formation of a melanin macromolecule.

Oxidizing agents that can achieve this goal comprise oxygen, hydrogen peroxide, ammonium persulfate, ferric ions, sodium iodide together with hydrogen peroxide, and treatment with a salt of a transition metal cation such as copper sulfate as a catalyst for air oxidation.

It is thus preferred when the oxidizing agent is chosen in the group consisting of oxygen, hydrogen peroxide (H₂O₂), ammonium persulfate, and ferric ions.

It is also preferred when the oxidative polymerization is performed in presence of tyrosinase.

In an embodiment, the synthetic melanin (post polymerization) is purified by filtration on a 5kDa-100kDa filter, preferably a 10kDa filter.

In a preferred embodiment, the synthetic melanin is a soluble melanin, i.e. is present in the form of particles of less than 500nm.

Thus, in an embodiment, the melanin is resuspended in water with or without buffer (such as phosphate buffer) prior to being mixed with the peptide.

### Obtaining the composition

An "immunogenic or immunostimulatory composition" is a composition that is able to generate an immune response in an animal when administered to said animal. Preferably, said animal is a mammal, but is can also be a bird (such as a chicken, a duck, a goose, a turkey, a quail), in particular when the composition is used in avian livestock. The animal may also be a fish, as the immunogenic composition may be used in fish farming. Such immunogenic or immunostimulatory composition is obtained when the peptide is an immunologically active peptide.

An immunogenic composition according to the invention is preferably used in mammals. Such mammals are preferably human beings, but can also be other mammals, when the composition is used in the veterinary field, in particular for inducing immunity in livestock such as cattle (cows), sheep, goats or horses, but also for pets such as dogs or cats.

The immunogenic composition is thus a composition that contains a peptide containing epitopes from an antigen, as disclosed above, and that is able to generate an immune response against such antigen. The generated immune response can be a cellular (T-cell mediated) or a humoral (B-cell mediated, production of antibodies) immune response. The immunogenic composition may thus induce both a cellular and a humoral immune response.

The cellular immune response can be a CD8 T lymphocytes mediated response (ie cytotoxic response), or a CD4 T lymphocytes mediated response (helper response). It can also combine a cytotoxic and helper cellular immune response. The helper response may involve Th1, Th2 or Th17lymphocytes (such lymphocytes being able to elicit different cytokine responses, as is known in the art).

The immunogenic composition may allow a better presentation of the antigen present therein, through MHC1 or MHC2 pathways.

The composition is obtained by adding a modified peptide, as disclosed above, to the synthetic melanin as herein described.

As an example, synthetic melanin can be obtained from a solution of L-Dopa incubated at pH 8.5+/-0.5 in aerobic conditions under agitation. Physico-chemical conditions can be modified to increase the reaction kinetics, such as increasing temperature above 20°C (for example between 60 and 80°C), of bubbling air into the reaction mixture, or increasing the atmospheric pressure. When synthetized, melanin is washed by filtration on an approximately 10kDa filter (melanin remains on the retentate), then resuspended in buffer (such as a phosphate buffer). Melanin can be filtered through a 0.2 µm filter for sterility. Peptides are then added to the melanin solution (weight ratio peptide/melanin between 1/1 and 1/10) and incubated for various periods of time before usage. The resulting solution can be washed and resuspended in water or in any appropriate buffer.

The binding of the peptide to the melanin can be verified by Tricine-SDS-PAGE analysis as described in Carpentier; 2017 (*op. cit*.). Briefly, samples (peptide-Mel or peptide alone) are loaded on acrylamide gels. Following electrophoresis, the gels are stained with Coomassie Brilliant Blue R-250, allowing the quantification of the free peptide in the gel. The binding of peptides to melanin can be expressed as the ratio: [amount of unbound peptide in samples Peptide-Mel / amount of peptides in control samples containing peptides alone.

### Addition of an adjuvant

The immunostimulatory composition as disclosed may also comprise another immunostimulatory molecule, ie an adjuvant as disclosed above.

An "adjuvant" is a substance that has the capacity to modify or enhance the immune response to an antigen. In other words, the immune response against the antigen may be higher or different in the presence of the adjuvant than when the adjuvant is not present (that includes when the response is modified, for example when the subset of T cells that are activated in the presence of the adjuvant is different from the subset activated in the absence of the adjuvant). Adjuvants are known in the art and have been widely used in the vaccine field.

One can cite alum, emulsions (either oil-in-water or water-in-oil, such as Freund's Incomplete Adjuvant (IFA) and MF59®), PRR (Pattern recognition receptors) Ligands, TLR3 (Toll-Like Receptor 3) and RLR (RIG-I Like Receptors) ligands such as double-stranded RNA (dsRNA), or synthetic analogs of dsRNA, such as poly(I:C), TLR4 ligands such as bacterial lipopolysaccharides (LPS), MPLA (monophosphoryl lipid A), in particular formulated with alum, TLR5 ligands such as bacterial flagellin, TLR7/8 ligands such as imidazoquinolines (i.e. imiquimod, gardiquimod and R848), TLR9 ligands such as oligodeoxynucleotides containing specific CpG motifs (CpG ODNs) or NOD2 (Nucleotide-binding oligomerization domain-containing protein 2) ligands. The term ligand above describes preferably an agonist of the receptor, *i.e.* a substance that binds to the receptor and activates the receptor.

It is preferred when this adjuvant is selected in the group consisting of TLR3 agonists and TLR9 agonists and in particular when this adjuvant that is further added is chosen among Polyinosinic:polycytidylic acid (poly I:C) and CpG oligonucleotides.

In a preferred embodiment, the adjuvant is added to the composition obtained just before administration, i.e. less than one hour before administration.

The invention also relates to an immunostimulatory composition susceptible to be obtained by a method herein disclosed. The invention also relates to an immunostimulatory composition obtained by a method herein disclosed.

Such composition can be distinguished from the compositions described in WO2017089529 (which are obtained by polymerization of the melanin precursor in presence of the antigen/peptide) in that the antigen has been added after the synthetic melanin was obtained rather than before oxidative polymerization.

### Use of the immunogenic composition

The invention also relates to the immunostimulatory composition as disclosed above for use thereof, as a vaccine to elicit an immune response against an antigen when administered to an animal (as disclosed above, including human being). Alternatively, the immunostimulatory composition can be used *in vitro* in presence of live cells (for example macrophages, dendritic cells or lymphocytes), to sensitize them to the antigen, for instance before administration (preferably injection) in humans or animal. The resulting composition will thus elicit an immune response against the antigen in the recipient. In particular, US 6210662 discloses such principle of forming therapeutic or immunogenic compositions consisting of antigen presenting cells activated by contact with an antigen complex. In the present case, the antigen-melanin complex is the one obtained according to methods described herein.

The invention also relates to the use of such an immunostimulatory composition to increase or elicit an immune response against a target antigen. This is particularly useful when the target antigen is not, by itself, immunogenic (*i.e.* no immune response is obtained when the antigen is administered alone).

In particular, binding the antigen to the synthetic melanin acts to increase the immune response to the antigen.

The invention also relates to an immunostimulatory composition as disclosed above, for its use as a vaccine to protect an animal against a disease implicating (*i.e.* involving and/or concerning) cells expressing inside the cells, at their surface, or secreting the target antigen or epitopes thereof.

The vaccine may be a prophylactic (*i.e.* intended to protect the recipient against the development of a disease) or a therapeutic (*i.e.* intended to help the recipient fight an already present disease) vaccine.

The protected animal has been disclosed above, and may be human being.

The disease is linked to the target antigen used in the immunostimulatory composition. This means that the antigen or an epitope thereof is expressed or presented by cells of the animal (or by pathogens) during the course of the disease. The disease thus involves or concerns cells expressing the target antigen. Such expression may be secretion of the antigen (as an illustration, the antigen may be a bacterial toxin), or surface expression of the antigen or epitope thereof (the antigen may be a surface protein of a virus, or an tumor-specific antigen or epitope thereof expressed at the surface of tumor cells), or presentation of the antigen or epitope thereof at the surface of cells (such as a MHC presentation of an antigen or epitope thereof by the target cell).

The invention also relates to a method for obtaining a medicament for treating a patient, comprising the steps of
a) Providing a synthetic melanin which has been obtained by an oxidative polymerization of a melanin precursor, and
b) Mixing it with a biologically/immunologically active peptide that has been modified by the addition of one or several amino-acids containing a nucleophilic residue
c) Optionally incubating the mixture
d) Optionally washing the mixture and resuspending the synthetic melanin which had bound the peptide,
thereby obtaining a drug for treating a patient (or an animal).

As a potential application, such formulation is able to elicit an immune response against the antigen (when the peptide is an antigen) when administered *in vivo,* or when incubated with cells *in vitro* (this would prime the cells which can then be administered to a patient or an animal).

The antigen used in this method is an antigen against which an immune response is sought in a recipient.

The synthetic melanin has been obtained by oxidative polymerization *in vitro.*

In a specific embodiment, the composition (used as a drug or a medicament) also contains an adjuvant, which is added prior to administration to the patient, either just before use or a few hours or days before use. Said adjuvant is other than a melanin precursor, and is preferably a TLR3 or TLR9 agonist, such as an adjuvant selected in the group consisting of poly I:C and CpG-oligonucleotides.

The invention also pertains to a method for eliciting an immune response against an antigen in a subject, comprising the step of administering a therapeutic or effective amount of an immunostimulatory composition as disclosed above to the subject, wherein the immunostimulatory composition has been obtained by mixing a synthetic melanin with the antigen or a peptide containing the antigen.

An "effective amount" or a "therapeutic" of an agent, as used herein, is the amount sufficient to induce beneficial or desired results, such as clinical results or onset of an immune response, in particular a T-cell mediated immune response. In the present context, a therapeutic amount of an agent is, for example, an amount sufficient to achieve onset of an immune response against the antigen, and reduction in the severity of a symptom of the disease linked to the antigen, as compared to the situation observed without administration of the composition. An effective amount is an amount that provides therapeutic improvement while minimizing side or adverse effect. One can use, as effective amounts, 10 µg to 5 mg of antigen, preferably between 100 µg and 500 µg. The amount of melanin that can be used may be comprised between 50 µg and 10 mg, in particular between 500 µg and 2 mg.

The invention also relates to a method for treating a patient in need thereof, comprising administering a therapeutic or effective amount of an immunostimulatory composition as disclosed herein to the patient, wherein said immunostimulatory composition induces an immune response against the antigen present in the immunostimulatory composition in said patient, and wherein the immune response has a therapeutic effect. The immune response may thus alleviate symptoms of the patient, reduce the load of a given pathogen, or to make a tumor, in particular a solid tumor, regress.

The invention also relates to a method for protecting a patient against a disease, comprising administering a therapeutic or effective amount of an immunostimulatory composition as disclosed herein to the patient, wherein said immunostimulatory composition induces an immune response against an antigen that is associated with the disease, wherein the immune response has a protective effect against the disease.

### Description of the Figures

Figure 1: CTL response after subcutaneous immunizations in 5-weeks old, C57BL/6, mice. Peptides (10 µg/mouse) were mixed with L-Dopa (weight ratio peptide/L-Dopa =1/4 and 1/6 for gp100 and EphA2 respectively) and incubated under the above described conditions (see table 1). Phosphorothioate oligonucleotide CpG-28 (5'-TAAACGTTATAACGTTATGACGTCAT, SEQ ID NO: 21), were added to vaccine formulations (10 µg/mouse) just before the immunizations. Mice were then immunized sub-cutaneously with gp100-Mel + CpG ("gp100-Mel"), or with previously synthesized melanin mixed with gp100 and CpG ("Mel + gp100"); EphA2-Mel + CpG ("EphA2-Mel"), melanin + EphA2 + CpG ("Mel + EphA2"). Mice were sacrificed on day 8 and the CTL response was performed as described in Carpentier; 2017. Briefly, splenocytes were re-stimulated in vitro with the corresponding MHC class I-epitope (non-conjugated to melanin) and the numbers of IFNg-SFCs (Spot forming cells) were measured and expressed as Mean+/-S.E.M. (n=8 mice/group with pooled data from 2 different experiments of 4 mice each. Student-T test: gp100-Mel vs Mel + gp100: p < 0.001; EphA2-Mel vs Mel + EphA2: p<0.001)
Figure 2: CTL response after subcutaneous immunizations in C57BL/6 mice. The gp100 peptide (10 µg/mouse) was mixed with L-Dopa (weight ratio peptide/L-Dopa =1/4 and incubated at pH 8.5 in aerobic conditions for 2 hours at 60°C to generate gp100-Mel. Alternatively, L-Dopa (0.8mg/ml) underwent an oxidative polymerization at pH 8.5 in aerobic conditions for 2 hours at 60°C. The reaction mixture was then filtered on a 10kDa filter, and the retentate containing the synthetic melanin was resuspended at pH 7.5 in phosphate buffer. Peptides (gp100 (SEQ ID NO: 1) or C-gp100 (SEQ ID NO: 17); 10 µg/mouse) were then added (at the weight ratio peptide/L-Dopa =1/4) and the mixtures (Mel + gp100 or Mel-C-gp100) were rapidly (<1 hour) used for subcutaneous immunizations in mice. Phosphorothioate oligonucleotide CpG-28 (5'-TAAACGTTATAACGTTATGACGTCAT, SEQ ID NO: 21) was added to vaccine formulations (10 µg/mouse) just before the immunizations. Mice were sacrificed on day 8 and the CTL response was performed as described in table 2. (n=12 mice/group with pooled data from 3 different experiments of 4 mice each. Student-T test: gp100-Mel vs Mel + gp100: p < 0.01; Mel + gp100 vs Mel + C-gp100: p<0.05)

### Examples

### Example 1. Preparing immunogenic compositions according to WO2017089529

Vaccine formulations combining antigens and synthetic melanin were prepared and tested for their ability to trigger cytotoxic T-lymphocyte (CTL) immune response (Carpentier et al, PLoS One. 2017 Jul 17;12(7):e0181403, WO2017089529). In these studies, short synthetic peptides (8-35 amino acids long) containing T-cell epitopes were mixed with a solution of L-Dopa, a precursor of melanin. The mixture was then oxidized to generate nanoparticles of melanin-bound peptides that can be efficiently used as a vaccine to trigger immune responses in mice. The binding of the antigens to synthetic melanin appeared critical to trigger immunity. Indeed, if the antigens (for ex the peptides gp100, EphA2) are added not before, but just after L-Dopa is polymerized in melanin, minimal binding of the peptides to melanin is seen in SDS-page analysis (Table 1), and the ability of the vaccine formulation to trigger a CTL (CD8) response in mice is lost (Figure 1).

**Table 1: Percentage of gp100 (KVPRNQDWL, SEQ ID NO: 1) or EphA2 (FSHHNIIRL, SEQ ID NO: 2) binding to melanin (Tricine-SDS-PAGE analysis).**

| | Peptide-Mel | Mel + peptide |
|---|---|---|
| KVPRNQDWL (SEQ ID NO: 1) | 100 +/-0% | 23 +/-8% |
| FSHHNIIRL (SEQ ID NO: 2) | 93 +/-12% | 9 +/-6% |

Peptides were mixed with L-Dopa (weight ratio peptide/L-Dopa =1/4 and 1/6 for gp100 and EphA2, respectively) then incubated for 2 hours at pH 8.5 and 60°C under agitation to ensure oxygenation of the solution to generate nanoparticles of melanin-bound peptides (Peptide-Mel). Alternatively, L-Dopa alone (0.8mg/ml) was polymerized into melanin for 2h at pH 8.5 and 60°C under agitation, before the peptides (weight ratio peptide/L-Dopa =1/4 and 1/6 for gp100 and EphA2, respectively) were added to the solution (Mel + peptide). Tricine-SDS-PAGE analysis was performed as described in Carpentier; 2017 (*op. cit*.). Briefly, samples (peptide-Mel or peptide alone) were loaded on acrylamide gels. Following electrophoresis, the gels were stained with Coomassie Brilliant Blue R-250 and imaged with the ChemiDoc XRS+ system (Bio-Rad Laboratory), allowing the quantification of the free peptide in the gel. The binding of peptides to melanin was expressed as the ratio: [amount of unbound peptide in samples Peptide-Mel / amount of peptides in control samples containing peptides alone]

### Example 2. Optimization of peptides binding to melanin

As shown above, efficient binding of the antigenic peptide to melanin plays a critical role to obtain biological properties. This binding that can be conveniently achieved by mixing the peptide during polymerization of L-Dopa as disclosed in WO2017089529.
Yet, concerns exist that peptides can be degraded during this oxidative process, which generates reactive oxygen species. A method of grafting/binding peptides on synthetic melanin (after synthetic melanin was obtained by oxidative polymerization of L-Dopa) was developed.

We first studied the radical moieties involved in melanin binding. Different peptides, all containing the basic sequence SIYRYYGL (SEQ ID NO: 3), were mixed with L-Dopa then incubated for 2 hours at pH 8.5 under agitation to generate nanoparticles of melanin-bound peptides (Peptide-Mel). As seen in the first column (Peptide-Mel) of table 3, melanin binding seems to depend upon the presence within peptides of nucleophilic moieties:
1) No binding was seen when the terminal NH2 was blocked by an acetyl residue (Acetyl-R-SIYRYYGL, SEQ ID NO: 4), pointing out the critical role of the -NH2-terminal end of peptides (and the minor role of the terminal -COOH moiety) in melanin binding.
2) Nucleophilic Proline or Hydroxyproline can be added at the NH2-terminal amino acid.
3) Lateral chain of some nucleophilic amino acids such as Lysine and Cysteine, also allowed a significant binding even when the NH2-terminal end of the peptides were blocked
Most surprisingly, even when L-Dopa alone was first polymerized into melanin, before the peptides were added to the solution, then incubated for 2 hours at room temperature (Mel + Peptide), some binding can be seen if some specific amino-acids (Cysteine, and to a minor degree Hydroxy Proline) are included in the peptide (Table 2, second column).

**Table 2: Binding of peptides on synthetic melanin (Tricine-SDS-PAGE analysis):**

| | | Peptide-Mel | Mel + peptide |
|---|---|---|---|
| Reference | SIYRYYGL (SEQ ID NO: 3) | **90%** | 0% |
| | Acetyl-R SIYRYYGL (SEQ ID NO: 4) | 0% | 17% |
| | | | |
| Nucleophile | P SIYRYYGL (SEQ ID NO: 5) | **87%** | 7% |
| | HydroxyP SIYRYYGL (SEQ ID NO: 6) | **95%** | **23%** |
| | | | |
| Nucleophile | Acetyl-R C SIYRYYGL (SEQ ID NO: 8) | **74%** | **53%** |
| | Acetyl-R H SIYRYYGL (SEQ ID NO: 9) | **60%** | ND |
| | | | |
| Non nucleophile | Acetyl-R T SIYRYYGL (SEQ ID NO: 12) | 10% | 0% |
| | Acetyl-R F SIYRYYGL (SEQ ID NO: 13) | 5% | 0% |
| | Acetyl-R W SIYRYYGL (SEQ ID NO: 14) | **56%** | 0% |

First column (Peptide-Mel): L-Dopa (0.8 mg/ml) was mixed with peptides (weight ratio peptide/L-Dopa =1/4) then incubated for 2 hours at pH 8.5 and 60°C to generate nanoparticles of melanin-bound peptides. Alternatively (second column; Mel + peptide), L-Dopa alone (0.8mg/ml) was first polymerized into melanin for 2h at pH 8.5 and 60°C under vigorous agitation, before the peptides (weight ratio peptide/L-Dopa =1/4) were added to the solution and incubated for 2 hours at room temperature. In both cases, the percentage of peptides that bound to melanin was then quantified with SPS-page analysis, as described in table 1. (ND=not done)

### Incubation conditions

We further investigated the impact on melanin binding of various incubations procedures. L-Dopa underwent oxidative polymerization; the reaction mixture was then filtered, and the retentate containing the synthetic melanin was then mixed with different peptides for various periods of time and temperature. Table 3 shows that limited binding of peptides is seen when the incubation time is short (approx. 10 minutes) as disclosed in the literature (Carpentier 2017). Yet, binding increased with incubation time, pH (table 3) or temperature (not shown). This binding is particularly seen when the peptide contained either a free NH2-terminal moiety, or one of the following amino acids: Lysine, Cysteine, Proline, HydroxyProline. Interestingly, for Cysteine, the impact of higher pH on melanin binding appeared limited.

**Table 3: Binding of peptides on synthetic melanin; impact of various physico-chemical conditions.**

| | | Mel + peptide 2 hours at pH 7,4 | Mel + peptide 18 hours at pH 7,4 | Mel + peptide 18 hours at pH 8,5 |
|---|---|---|---|---|
| reference | SIYRYYGL (SEQ ID NO: 3) | 0% | 28% | 31% |
| | | | | |
| nucleophile | **P** SIYRYYGL (SEQ ID NO: 5) | 7% | 22% | **41%** |
| | **hydroxyP** SIYRYYGL (SEQ ID NO: 6) | 23% | 39% | **74%** |
| | | | | |
| nucleophile | Acetyl-**K** SIYRYYGL (SEQ ID NO: 7) | 0% | 31% | **62%** |
| | Acetyl-R **C** SIYRYYGL (SEQ ID NO: 8) | **53%** | **46%** | **42%** |
| | Acetyl-R **S** SIYRYYGL (SEQ ID NO: 10) | 0% | 12% | ND |
| | Acetyl-R **M** SIYRYYGL (SEQ ID NO: 11) | 0% | 13% | 32% |
| | | | | |
| non nucleophile | Acetyl-**R** SIYRYYGL (SEQ ID NO: 4) | 17% | 2% | 0% |
| ID | Acetyl-R **T** SIYRYYGL (SEQ ID NO: 12) | 0% | ND | ND |
| | Acetyl-R **F** SIYRYYGL (SEQ ID NO: 13) | 0% | ND | ND |
| | Acetyl-R **W** SIYRYYGL (SEQ ID NO: 14) | 0% | ND | ND |

L-Dopa (0.8mg/ml) underwent an oxidative polymerization at pH 8.5 in aerobic conditions for 2 hours at 60°C. The reaction mixture was then filtered on a 10kDa filter, and the retentate containing the synthetic melanin was resuspended at pH 7.5 in phosphate buffer. Peptides were then added (at the weight ratio peptide/L-Dopa =1/4) and the mixture was incubated for various periods of time and or pH. The percentage of peptides that bound to melanin was then quantified with SPS-page analysis, as described in table 1. (ND=not done).

A similar experiment was carried out on a family of gp100 peptides (basic sequence: KVPRNQDWL (SEQ ID NO: 1), see also Example 1): (Table 4). Again, when melanin is synthetized before the peptides are added, the melanin binding of peptides is enhanced 1) by increasing the incubation time, and b) when peptides contained specific amino acids such as Lysine, Cysteine, hydroxyproline or methionine.

**Table 4: Binding of peptides on synthetic melanin; impact of incubation time and amino-acids.**

| | | Mel + peptide 10 minutes | Mel + peptide 2 hours | Mel + peptide 18 hours |
|---|---|---|---|---|
| reference | KVPRNQDWL (SEQ ID NO: 1) | 6% | 22% | 89% |
| | | | | |
| nucleophile | P KVPRNQDWL (SEQ ID NO: 15) | 13% | 11% | 100% |
| | HydroxP KVPRNQDWL (SEQ ID NO: 16) | 12% | **35%** | 100% |
| | C KVPRNQDWL (SEQ ID NO: 17) | 9% | **54%** | 99% |
| | M KVPRNQDWL (SEQ ID NO: 18) | 7% | **43%** | 100% |

L-Dopa (0.8mg/ml) underwent an oxidative polymerization at pH 8.5 and 60°C for 2 hours. The reaction mixture was then filtered on a 10kDa filter, and the retentate containing the synthetic melanin was resuspended at pH 7.5 in phosphate buffer. Peptides were then added (at the weight ratio peptide/L-Dopa =1/4) and the mixture was incubated for various periods of time. Binding of the peptides was then quantified with SPS-page analysis, as described in table 1. (ND=not done)

Similar results were also obtained with various peptides of different length: adding a Cysteine at the NH2-terminal end of the peptide significantly increased the binding on synthetic melanin (data not shown).

When the amino-acid promoting the melanin binding was placed at the COOH terminal end, instead of the NH2-terminal end of a peptide containing an epitope (for example X-VYDFFVWL (SEQ ID NO: 19) vs VYDFFVWL-X (SEQ ID NO: 20)), the peptide binding to melanin was similar (51% vs 55%).

As Cysteine can be either in a oxidized or reduced states, we studied its binding to melanin in both cases. L-Dopa underwent oxidative polymerization; the reaction mixture was then filtered, and the retentate containing the synthetic melanin was then mixed with the Cgp100 (SEQ ID NO: 17) (either in a oxidized or reduced state) peptide for 2 hours at room temperature. While the binding was 79% in the reduced state, reduced binding was seen when cysteine was in the oxidized state (25%).

Finally, we checked that one of the above-described formulations in which peptides had a good melanin binding (> 50%) has a good biological activity. As shown in Figure 2, immunization of mice with one of such formulations induced an immune response of the same magnitude as historical controls in which peptides were mixed to L-Dopa before oxidative polymerization.

### Conclusion

Altogether, these experiments show that addition of nucleophilic moieties contained in amino-acids such as Cysteine, Proline, hydroxyProline, Lysine, Methionine to peptides increases binding thereof to melanin. The immunological efficacy of these formulations are similar to the one disclosed in the prior art (WO2017089529), where peptides are added in the reaction mixture before L-Dopa got oxidized.
The co-incubation of peptides with a previously synthetized melanin, when a nucleophilic amino acid has been added to the peptide is thus an efficient way to increase binding to melanin, and the immune response resulting therefrom. It also presents the advantage of the better control of both the quality of the synthetic melanin (which can be reliably characterized, which is an advantage for regulatory matters) and the quantity of the peptide actually bound to the melanin. This process also provides a better protection of the integrity of the peptide than the one of the prior art, where such peptide is submitted to the oxidizing agent used for polymerizing the melanin precursor.

## Claims

1. A method for obtaining a composition, comprising the steps of
a) Providing a synthetic melanin which has been obtained by an oxidative polymerization of a melanin precursor, and
b) Mixing it with a peptide that has been modified by the addition of one or several amino-acids containing a nucleophilic residue, so as to obtain a composition comprising melanin bound to the modified peptide.

2. The method according to claim 1, wherein the amino-acid added to the peptide is selected from the group consisting in cysteine, acetylcysteine, methionine, proline, hydroxyproline, histidine and lysine.

3. The method according to claim 2, wherein the amino-acid peptide has been added at the N-terminus of the peptide.

4. The method according to any one of claims 1 to 3, wherein the peptide modified by addition of one or several amino-acids containing a nucleophilic residue is selected from the group consisting of SEQ ID NO: 22, SEQ ID NO: 23, SEQ ID NO: 24, SEQ ID NO: 25, SEQ ID NO: 26 and SEQ ID NO: 27.

5. The method according to any one of claims 1 to 3, wherein the melanin precursor is selected from the group consisting of DHICA, DHI, L-dopa, L-tyrosine, D-dopa, 6-hydroxy-Dopa, dopaquinone, cyclodopa, dopachrome, dopamine-o-quinone, dopamine, leukodopaminochrome and dopaminochrome.

6. The method according to claim 4, wherein the melanin precursor is L-Dopa.

7. The method according to any one of claims 1 to 6, wherein the oxidative polymerization is performed in presence of oxygen, H₂O₂ or persulfate as oxidizing agents as the oxidizing agent.

8. The method according to any one of claims 1 to 7, wherein the oxidative polymerization is performed in presence of tyrosinase.

9. The method according to any one of claims 1 to 8, wherein the synthetic melanin is first purified by filtration on a 10kDa filter, before being mixed with the peptide

10. The method of any one of claims 1 to 9, wherein an immune adjuvant is added to the composition obtained after step b) or before administration.

11. An immunostimulatory composition susceptible to be obtained by a method according to any one of claims 1 to 10.

12. The immunostimulatory composition of claim 11, for its use as a vaccine to protect a human or an animal against a disease implicating cells expressing an antigen expressing the epitope.

13. The immunostimulatory composition for its use according to claim 12, wherein the disease is cancer, a virus infection, a bacterial infection, a fungus infection or a parasite infection.

14. The immunostimulatory composition for its use according to claim 13, wherein the disease is a low or high grade glial tumor.


SEQUENCE LISTING

<110>  ASSISTANCE PUBLIQUE HÔPITAUX DE PARIS
       ALTEVAX

<120>  OPTIMIZATION OF PEPTIDE-MELANIN BINDING

<130>  BRV 160-EP PRIO

<160>  27

<170>  PatentIn version 3.5

<210>  1
<211>  9
<212>  PRT
<213>  Artificial

<220>
<223>  gp100 peptide

<400>  1

Lys Val Pro Arg Asn Gln Asp Trp Leu
1               5


<210>  2
<211>  9
<212>  PRT
<213>  Artificial

<220>
<223>  EphA2 peptide

<400>  2

Phe Ser His His Asn Ile Ile Arg Leu
1               5


<210>  3
<211>  8
<212>  PRT
<213>  Artificial

<220>
<223>  test peptide

<400>  3

Ser Ile Tyr Arg Tyr Tyr Gly Leu
1               5


<210>  4
<211>  9
<212>  PRT
<213>  Artificial

<220>
<223>  Modified test peptide

<220>
<221>  MOD_RES
<222>  (1)..(1)
<223>  ACETYL-ARGININE

<400>  4

Arg Ser Ile Tyr Arg Tyr Tyr Gly Leu
1               5


<210>  5
<211>  9
<212>  PRT
<213>  Artificial

<220>
<223>  Modified test peptide

<400>  5

Pro Ser Ile Tyr Arg Tyr Tyr Gly Leu
1               5


<210>  6
<211>  9
<212>  PRT
<213>  Artificial

<220>
<223>  Modified test peptide


<220>
<221>  MOD_RES
<222>  (1)..(1)
<223>  HYDROXY-PROLINE

<400>  6

Pro Ser Ile Tyr Arg Tyr Tyr Gly Leu
1               5


<210>  7
<211>  9
<212>  PRT
<213>  Artificial

<220>
<223>  Modified test peptide


<220>
<221>  MOD_RES
<222>  (1)..(1)
<223>  ACETYL-LYSINE

<400>  7

Lys Ser Ile Tyr Arg Tyr Tyr Gly Leu

1               5


<210>  8
<211>  10
<212>  PRT
<213>  Artificial

<220>
<223>  Modified test peptide



<220>
<221>  MOD_RES
<222>  (1)..(1)
<223>  ACETYL-ARGININE

<400>  8

Arg Cys Ser Ile Tyr Arg Tyr Tyr Gly Leu
1               5                   10


<210>  9
<211>  10
<212>  PRT
<213>  Artificial

<220>
<223>  Modified test peptide



<220>
<221>  MOD_RES
<222>  (1)..(1)
<223>  ACETYL-ARGININE

<400>  9

Arg His Ser Ile Tyr Arg Tyr Tyr Gly Leu
1               5                   10


<210>  10
<211>  10
<212>  PRT
<213>  Artificial

<220>
<223>  Modified test peptide



<220>
<221>  MOD_RES
<222>  (1)..(1)
<223>  ACETYL-ARGININE

<400>  10

Arg Ser Ser Ile Tyr Arg Tyr Tyr Gly Leu
1               5                   10

<210>  11
<211>  10
<212>  PRT
<213>  Artificial

<220>
<223>  Modified test peptide


<220>
<221>  MOD_RES
<222>  (1)..(1)
<223>  ACETYL-ARGININE

<400>  11

Arg Met Ser Ile Tyr Arg Tyr Tyr Gly Leu
1               5                   10


<210>  12
<211>  10
<212>  PRT
<213>  Artificial

<220>
<223>  Modified test peptide


<220>
<221>  MOD_RES
<222>  (1)..(1)
<223>  ACETYL-ARGININE

<400>  12

Arg Thr Ser Ile Tyr Arg Tyr Tyr Gly Leu
1               5                   10


<210>  13
<211>  10
<212>  PRT
<213>  Artificial

<220>
<223>  Modified test peptide


<220>
<221>  MOD_RES
<222>  (1)..(1)
<223>  ACETYL-ARGININE

<400>  13

Arg Phe Ser Ile Tyr Arg Tyr Tyr Gly Leu
1               5                   10


<210>  14
<211>  10
<212>  PRT

<213>  Artificial

<220>
<223>  Modified test peptide

<220>
<221>  MOD_RES
<222>  (1)..(1)
<223>  ACETYL-ARGININE

<400>  14

Arg Trp Ser Ile Tyr Arg Tyr Tyr Gly Leu
1               5                   10

<210>  15
<211>  10
<212>  PRT
<213>  Artificial

<220>
<223>  Modified gp100 peptide

<400>  15

Pro Lys Val Pro Arg Asn Gln Asp Trp Leu
1               5                   10

<210>  16
<211>  10
<212>  PRT
<213>  Artificial

<220>
<223>  Modified gp100 peptide

<220>
<221>  MOD_RES
<222>  (1)..(1)
<223>  HYDROXY-PROLINE

<400>  16

Pro Lys Val Pro Arg Asn Gln Asp Trp Leu
1               5                   10

<210>  17
<211>  10
<212>  PRT
<213>  Artificial

<220>
<223>  Modified gp100 peptide

<400>  17

Cys Lys Val Pro Arg Asn Gln Asp Trp Leu
1               5                   10

<210>  18
<211>  10
<212>  PRT
<213>  Artificial

<220>
<223>  Modified gp100 peptide

<400>  18

Met Lys Val Pro Arg Asn Gln Asp Trp Leu
1               5                   10


<210>  19
<211>  9
<212>  PRT
<213>  Artificial

<220>
<223>  N-terminus Modified peptide


<220>
<221>  VARIANT
<222>  (1)..(1)
<223>  Xaa IS A  NUCLEOPHILIC AMINO ACID, ESPECIALLY CYSTEINE, PROLINE
       OR HYDROXYPROLINE

<400>  19

Xaa Val Tyr Asp Phe Phe Val Trp Leu
1               5


<210>  20
<211>  9
<212>  PRT
<213>  Artificial

<220>
<223>  C-terminus Modified peptide


<220>
<221>  VARIANT
<222>  (9)..(9)
<223>  Xaa IS A  NUCLEOPHILIC AMINO ACID, ESPECIALLY CYSTEINE, PROLINE
       OR HYDROXYPROLINE

<400>  20

Val Tyr Asp Phe Phe Val Trp Leu Xaa
1               5


<210>  21
<211>  26
<212>  DNA
<213>  Artificial

<220>
<223>  Phosphorothioate oligonucleotide CpG-28

<400>  21
taaacgttat aacgttatga cgtcat                                            26


<210>  22
<211>  16
<212>  PRT
<213>  Homo sapiens

<400>  22

Tyr Ala Val Gly Tyr Met Leu Arg Leu Gly Ala Pro Ala Ser Lys Leu
1               5                   10                  15


<210>  23
<211>  16
<212>  PRT
<213>  Homo sapiens

<400>  23

Thr Thr Met Asp Gln Lys Ser Leu Trp Ala Gly Val Val Val Leu Leu
1               5                   10                  15


<210>  24
<211>  15
<212>  PRT
<213>  Homo sapiens

<400>  24

Lys Ser Val Trp Ser Lys Leu Gln Ser Ile Gly Ile Arg Gln His
1               5                   10                  15


<210>  25
<211>  15
<212>  PRT
<213>  Human coronavirus

<400>  25

Tyr Val Gly Tyr Leu Gln Pro Arg Thr Phe Leu Leu Lys Tyr Asn
1               5                   10                  15


<210>  26
<211>  11
<212>  PRT
<213>  Human coronavirus

<400>  26

Gly Tyr Leu Gln Pro Arg Thr Phe Leu Leu Lys
1               5                   10


<210>  27

<211>  10
<212>  PRT
<213>  Human coronavirus

<400>  27

Lys Val Val Asn Gln Asn Ala Gln Ala Leu
1               5                   10